# EUROPEAN PATENT SPECIFICATION

(11) **EP 0 614 382 B1**
(45) Date of publication and mention of the grant of the patent: **13.03.1996**
(21) Application number: 92922414.5
(22) Date of filing: 19.10.1992
(51) Int. Cl.: A61M 5/172, H05K 5/00

(54) **INTERLOCKING MODULE SYSTEM**
INEINANDERGREIFENDER MODULENSYSTEM
SYSTEME DE MODULES A VERROUILLAGE RECIPROQUE

(30) Priority: 25.11.1991 US 797691
(43) Date of publication of application: 14.09.1994
(73) Proprietor: MINNESOTA MINING AND MANUFACTURING COMPANY, St. Paul, Minnesota 55133-3427 (US)
(72) Inventor: ANDERSON, Jerald, W., Saint Paul, MN 55133-3427 (US); GRAMSE, Leonard, J., Saint Paul, MN 55133-3427 (US); OJA, Gerald, L., Saint Paul, MN 55133-3427 (US); STRUBLE, Kent, R., Saint Paul, MN 55133-3427 (US); TOYCEN, Mark, A., Saint Paul, MN 55133-3427 (US)
(74) Representative: Hilleringmann, Jochen, Dipl.-Ing.
(86) International application number: US9208899
(87) International publication number: WO9310835

(56) References cited:
- DE-U- 9 000 646
- US-A- 4 535 870
- US-A- 4 637 749
- US-A- 4 756 706

## Description

The invention relates generally to a system for mounting modules in side-by-side relation at one another, and more particularly to a system for mounting modules, such as medical instrument modules, on a pole stand.

In modern medical practice a variety of diagnostic and therapeutic instruments are used, sometimes to such a degree that floor and shelf space near the patient's bedside is at a premium. One known solution to the problem of mounting instrument modules is the use of a pole stand, which allows equipment to be mounted vertically over a relatively small footprint on the floor. Often such pole stands have wheels for the convenience of the operator in moving them to where they are needed. It is a well known problem in the hospital setting that such wheeled stands are easily unbalanced upon, for example, crossing thresholds or exiting elevators.

A related concern is matter of rapidly mounting and demounting various instruments in a hospital setting. In a crisis situation, it can be important that the proper mounting of an instrument module be accomplished expeditiously. Even in routine operations, a convenient and reliable mounting system would increase efficiency while enhancing patient comfort. In particular, infusion therapy is performed on many patients in the hospital, and due to incompatibilities in drug chemistry or infusion regimen, this often requires several infusion lines. Over the course of a hospital stay, the number of infusion lines, and hence the number of infusion pumps managing the flow within them, is likely to vary. The cost of patient care is reduced if the infusion pumps can rapidly be redeployed where needed.

Examples of infusion pumps that have typically been mounted on pole stands are shown in U.S. Patent No. 5,017,192 and U.S. Design Patent No. 278,181. Typically, a single infusion pump has been mounted on the pole stand at one vertical location along the stand. If additional infusion pumps were needed, they would be mounted on separate pole stands, or at a different vertical location along the same pole stand, or on specially designed multi-pole adaptors or stands that provide a plurality of spaced apart vertical pole sections for mounting infusion pumps. Such infusion pumps have been available from Minnesota Mining and Manufacturing Company, St. Paul, Minnesota, under various trade designations, such as the "AVI 200", "AVI 400" and "AVI 480" model series.

Dual channel infusion pumps have also been available in which one pump housing contains two separately controllable infusion pumps. Dual channel infusion pumps have been available from Minnesota Mining and Manufacturing Company, St. Paul, Minnesota, under the trade designations "AVI 840" and "AVI MICRO 845". In the case of dual channel infusion pumps, space on the pole stands is economized by mounting one housing on the pole stand to provide two pumps and two flow channels. Another dual channel infusion pump is available from IMED Corporation, San Diego, California, under the trade designation "GEMINI PC2". Of course, one disadvantage of any dual channel infusion pump is that in effect two infusion pumps are provided even in situations where only one pump is needed.

Specially designed pole stands have been used to support a plurality of infusion pumps. One such pole stand has been sold by Minnesota Mining and Manufacturing Company, St. Paul, Minnesota, under the trade designation "Model 166 Multipump IV Pole". The "Model 166" pole stand has a lower single pole section supporting an upper multiple pole section, which comprises a plurality of spaced apart pole sections. The upper pole sections of that pole stand have been used to support more than one infusion pump at the same vertical level. Specially designed adaptors have also been available to provide more than one spaced apart vertical pole section on a single pole stand. An adaptor of that type has been available from Minnesota Mining and Manufacturing Company under the trade designation "Model 146 IV Pole Adaptor".

U.S. Patent Nos. 4,756,706 and 4,898,578 show a drug infusion system with calculator. That system includes a plurality of infusion pump modules arranged in a vertical stack along with a central management unit, all of which are supported on a single pole stand. The modules are electronically interlinked with the central management unit. Each module comprises a frame having opposite sides and interlocking means on the frame adapted for attaching at least two other modules of the system to the module. U.S. Patent No. 4,356,475 (Neumann et al.) shows a system containing a predetermined number of monitoring devices and at least one central station. That system includes equipment having a bay into which slide-in modules can be introduced in levels one above the other.

It is the object of the invention, to provide a system for interlocking a plurality of modules in side-by-side relationship avoiding the modules supporting means from being unbalancedly loaded and/or overloaded excessively.

The invention provides a system of interconnecting of modules, such as medical device modules (e.g., an infusion pump), such that one module (designated the "center" module) can have two modules (designated the "side" modules) connected on opposite sides of the center module but that prevents additional modules from being mounted on the side modules. The modules of the invention are designed to be interchangeable in so far as the system is concerned, i.e., a "side" module of one set-up can be used as a "center" module of another set-up and visa versa. The invention also provides such a system in which the act of attaching the "center" module to a pole stand automatically allows the attachment of "side" modules to the "center" module, which the "center" module would prevent absent its attachment to the pole stand.

Generally, a system of the invention is adapted for interlocking a plurality interchangeable modules in side-by-side relationship. The modules of the invention each generally comprises a frame having opposite sides, and interlocking means on the frame adapted for attaching at least two other modules of the system to the module. The interlocking means includes condition-setting means for setting the interlocking means in a first condition for mounting the module on another module of the system or a second condition for mounting other module(s) of the system thereon. The arrangement is such that (a) when the interlocking means is set in its first condition the interlocking means is capable of being mounted on the interlocking means of another module of the system that has been set in its second condition but the interlocking means of the module is not capable of having mounted thereon the interlocking means of another module of the system, and (b) when the interlocking means is set in its second condition it is capable of having mounted thereon the interlocking means of two other modules of the system that have been set in their first condition.

Preferably, each module includes securing means for securing the module to a pole, and automatic means for setting the module in its second condition upon securing the module to a pole via the securing means.

In one alternative aspect of the invention, the interlocking means of each module comprises a pair of generally L-shaped support bodies rotatably mounted on opposite sides of the module. Each of the support bodies has two legs including one leg comprising a male dovetail-type slide connection adapted for connection with a complementary female dovetail-type slide connection of another module, and another leg comprising a female dovetail-type slide connection adapted for connection with a complementary male dovetail-type slide connection of another module. In this alternative, the condition-setting means comprises means for pivotably mounting the support bodies on opposite sides of the module, with the male dovetail-type slide connections of the module being aligned in one direction and the female dovetail-type slide connections of the module being aligned in another direction generally perpendicular to the direction of the male dovetail-type slide connections. The support bodies are pivotable between a first position, wherein one aligned pair of the male or female dovetail-type slide connections are aligned in the vertical direction, and a second position, wherein the other aligned pair of the male or female dovetail-type slide connections are aligned in the vertical direction.

In one preferred aspect of the invention, the module is in the form of a medical instrument module, such as an infusion pump, that is adapted for use in an interlocking system of similar medical instrument modules that are adapted to be mounted in side-by-side relationship on a pole stand. The module generally comprises a frame having opposite sides, securing means for securing the module to a pole, and at least two slide connections mounted on opposite sides of the module. The slide connection on one side of the module is complementary to the slide connection on the other side of the module, and the slide connections are arranged along the module such that the direction of slide in the connection is generally along a vertical axis when the module is in use. At least two latching surfaces are provided on the module, each corresponding to one of the slide connections. The latching surfaces are adapted for engaging a latching arm of a similar module when the slide connection of the module is interconnected with a complimentary slide connection of the similar module. A latching arm is movably mounted in the frame, and is adapted for engaging a latching surface on a similar module to enable the module to be mounted on the similar module. More specifically, the latching arm is movable between a first position and a second position. In the first position, the latching arm extends relative to the latching surfaces and slide connections a distance sufficient to engage the shoulder of a similar module when one of the slide connections are mounted on the slide connection of the similar module and wherein the latching arm blocks the slide connection from having a slide connection of a similar module mounted thereon. In the second position, the latching arm is withdrawn relative to said latching surfaces and slide connections to allow a slide connection of a similar module to be mounted on said slide connection.

Preferably, at least two latching arms are provided at opposite sides of said module, with each latching arm corresponding to one of said slide connections. Each arm includes a rack portion having gear teeth. The module also includes a gear rotatably mounted on the frame, with the gear teeth of the gear in intermeshing relationship with the gear teeth of the arms such that rotation of the gear in one direction withdraws the arms from the first position to their second position. Biasing means, such a spring, may also be provided for biasing said arms toward their first position. A tongue on one of the arms extends into the securing means such that when the tongue is engaged by a pole to which the securing means is secured, the tongue is moved by the pole to move the arms to their second position.

Other features will be in part apparent and in part pointed out hereinafter.

The invention will be further described with reference to the drawing wherein corresponding reference characters indicate corresponding parts throughout the several views of the drawing, and wherein:
Figure 1 is a frontal perspective view of three modules of the preferred embodiment of the invention illustrating their being mounted on a single pole stand;
Figure 2 is a top plan view of the three modules of figure 1 mounted on the single pole stand;
Figure 3 is a partial cross-sectional view substantially along line 3-3 of figure 2 illustrating a first condition of the module in which the module is adapted to be mounted on the side of another module;
Figure 4 is a modified cross-sectional view similar to figure 3 illustrating a second condition of the module in which the module is adapted to have other modules set in their first condition mounted on its opposite sides;
Figure 5 is an enlarged exploded diagrammatic top view of complementary slide connections on the modules taken at area 5-5 of figure 2 illustrating details of slide connections that are adapted to help interlock adjacent modules together;
Figure 6 is an enlarged diagrammatic view of a portion 6-6 of two interlocked modules of figure 4 illustrating details of the arrangement when one module is mounted on another;
Figure 7A and 7B are side views of a portion of outer sides the two side modules shown as illustrated in figure 2;
Figure 8 is an enlarged diagrammatic view of selected portions of figure 7A and 7B illustrating various details of the mechanism for preventing mounting of more than one side module on one side of a center module;
Figure 9 is a frontal perspective view of two modules of an alternative embodiment of the invention; and
Figure 10 is a cross-sectional view substantially along line 10-10 in figure 9 illustrating details of interlocking slide connections of that embodiment.

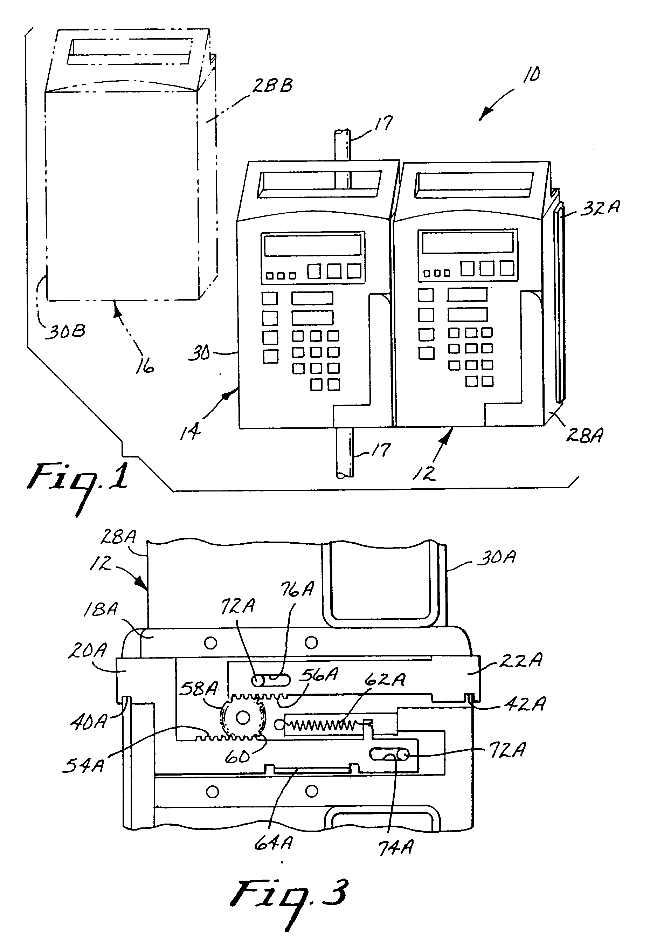

As illustrated in figure 1, the system 10 of the invention comprises a plurality of interchangeable modules 12, 14 and 16 which are adapted to be releasably interlocked in a side-by-side arrangement supported by one of the modules 14 on a pole stand 17.

The modules 12, 14 and 16 may be in the form of three infusion pumps 12, 14 and 16 of the general type described in U.S. Patent Nos. 4,236,880; 4,277,226 4,322,201; and 5,017,192, as well as U.S. Patent Application Serial No. 07/690,819, filed April 23, 1991 by Thill, Toycen, Struble and Curran. One or more of the modules 12, 14 and 16 may also be of the general type sometimes referred to as "syringe pumps", such as described in U.S. Patent Nos. 4,202,333; 4,298,000; 4,430,079 and 4,597,754. It is also contemplated that the modules 12, 14 and/or 16 of the invention might include other types of modules, such as other medical device modules. Examples include patient monitoring equipment, as well as other types of modules that may be employed in medical procedures.

As used herein, the term "interchangeable" refers to the feature of the modules 12, 14 and 16 being capable of being arranged in each other's position. For example, either one of the "side" modules 12 and 16 in figures 1 and 2 could be repositioned in the center or exchanged with each other, and the "center" pump 14 could be positioned at either side. "Interchangeable" is not intended to refer the function of the modules 12, 14 and 16, e.g., one module could be an infusion pump, and another module of the same system could be a patient monitor.

The modules 12, 14 and 16 illustrated in the drawings are substantially identical, and will first be described with reference to the center module 14 and right side module 12. Corresponding features in the "right side" module 12 and "left side" module 16 are indicated by the same reference numerals as the "center" module 14 but are followed by an "A" or "B" respectively. Regardless of which module 12, 14 or 16 is being described, the relevant features of the other modules of the preferred embodiment are identical.

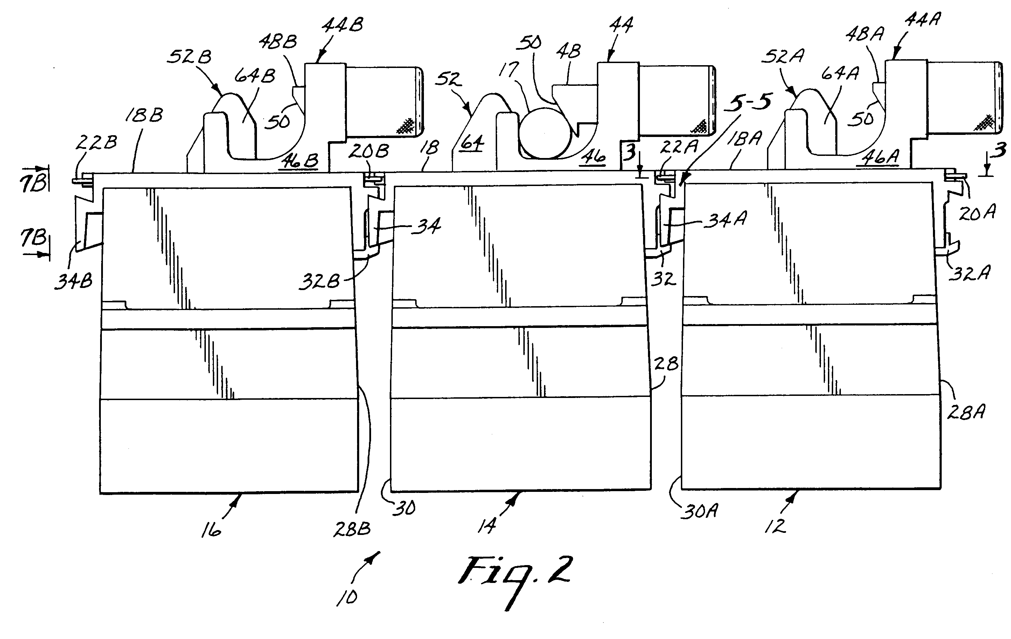

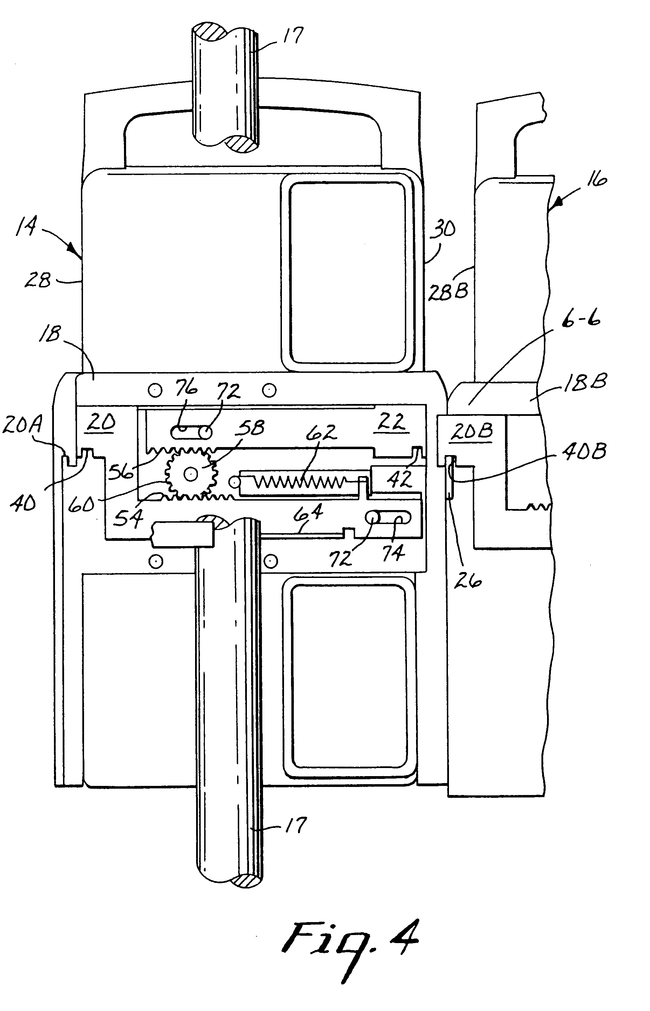

Module 14 generally comprises a frame 18, and interlocking means on the frame 18 for attaching at least two similar modules (e.g., 12 and 16) having interlocking means to the module 14. The interlocking means includes condition-setting means (e.g., latching arms 20 and 22) for setting the interlocking means in a first condition (figure 3) or a second condition (figure 4). As illustrated with "right" and "left" modules 12 and 16 in figures 1-3, in the first condition, the interlocking means is adapted for mounting the module 12 or 16 on a similar module 14. For example, the latching arms 20A and 22A of module 12 are shown in their extended position in figure 3, and one of the latching arms 20B of module 16 is shown in its extended position in figure 4. As illustrated with "center" module 14 in figures 1-2 and 4, in the second condition, the interlocking means is adapted for mounting similar module(s) (e.g., 12 and 14) thereon. For example, the latching arms 20 and 20A of the center module 14 are shown in their retracted position in figure 4.

The arrangement is such that, when the interlocking means of a module (e.g., 12) is set in its first condition, the interlocking means is capable of being mounted on the interlocking means of a similar module (e.g., 14) that has been set in its second condition, but the interlocking means of the module 12 is not capable of having mounted thereon the interlocking means of a similar module (e.g., 16) that has been set in its first condition. The arrangement is also such that, when the interlocking means of a module (e.g., 14) is set in its second condition, it is capable of having mounted thereon the interlocking means of at least two similar modules (e.g., 12 and 14) that have been set in their first condition. Various alternatives as to how this can be accomplished will be described below.

For purposes of illustration, the arrangement in the first condition will be described mostly with respect to "right" module 12, and the arrangement in the second condition will be described mostly with respect to "center" module 14. In the preferred embodiment illustrated in figures 1-8, the difference between the first and second conditions is whether the latching arms 20 and 22 are extended as illustrated at 20A and 22A in figure 3 or retracted as illustrated at 20 and 22 in figure 4.

In the first condition, as shown in figure 3 the latching arms 20A and 22A of module 12 are extended to a first position, or as shown in figure 4 the latching arms 208 of module 16 are extended to the first position, and with either module 12 or 16 the intended result being to latch onto a corresponding latching surface 24 or 26 of the center module 14 (with corresponding latching arms 20A and 22B) to mount module 12 and 16 on the right or left side 28 or 30 of module 14. In the second condition, the latching arms 20 and 22 of module 14 are retracted to a second position (figure 4).

Preferably, at least two slide connections 32 and 34 are provided on opposite sides 28 and 30 of the module 14. The slide connection 32 on one side 28 of each module 14 is complementary to the slide connection 34 on the other side 30 of the module 14. The arrangement is such that the slide connection 32, 32A or 32B on the right side 28 of any module 12, 14 or 16 of one compatible system 10 is complementary to the slide connection 34, 34A or 34B on the left side 30 of any other module 12, 14 or 16 of the same system 10.

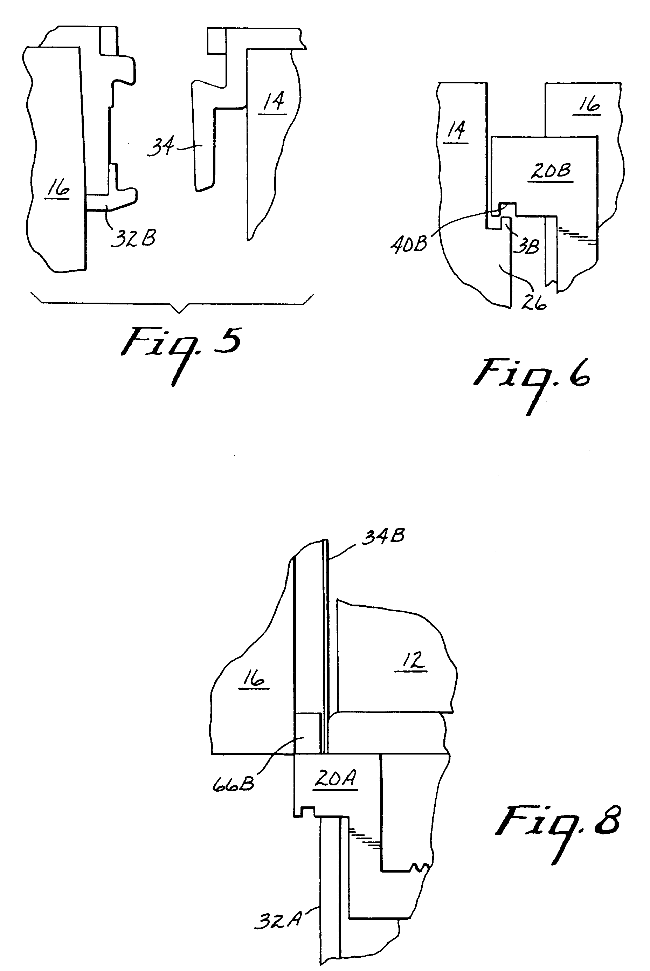

As used herein, the term "complementary" refers to the feature of the slide connections on opposite sides 28 and 30 of the modules being compatible with one another so as to properly fit each other to interchangeably mount the modules 12, 14 and 16 on one another. For example, as illustrated in figures 2 and 5, the slide connections 32, 32A and 32B on the right sides 28, 28A and 28B of the modules 14, 12 and 16, respectively, may comprise female dovetail-type slide connections 32, 32A and 32B, and the slide connections 34, 34A and 34B on the left sides 30, 30A and 30B may comprise "complementary" male dovetail-type slide connections 34, 34A and 34B. The male dovetail-type slide connections 34, 34A and 34B are adapted to be securely engaged in any of the female dovetail-type slide connections 32, 32A and 32B. Figure 5 is a diagrammatic top view of area 5-5 in figure 2 illustrating that the male dovetail-type slide connections 34A are sized to be closely received in the open area of the female dovetail-type slide connections 32.

As illustrated in figures 1, 2 and 7A-B, the slide connections, e.g., 32A, are arranged along the modules 12, 14 and 16 such that the direction of slide in the connection 32, 32A, 32B, 34, 34A and 34B is generally along a vertical axis when the modules 12, 14 and 16 are in use. This orientation of the slide connections 32, 32A, 32B, 34, 34A and 34B facilitates mounting the side modules 12 and 16 on the center module 14. The side modules 12 and 16 merely have to be raised relative to the center module 14, and the lower end of the appropriate slide connection 34A and 32B of the side modules 12 and 16 slide into or around the complementary slide connection 32 and 34, respectively, of the center module 14 to mount the side modules 12 and 16 on the center module 14.

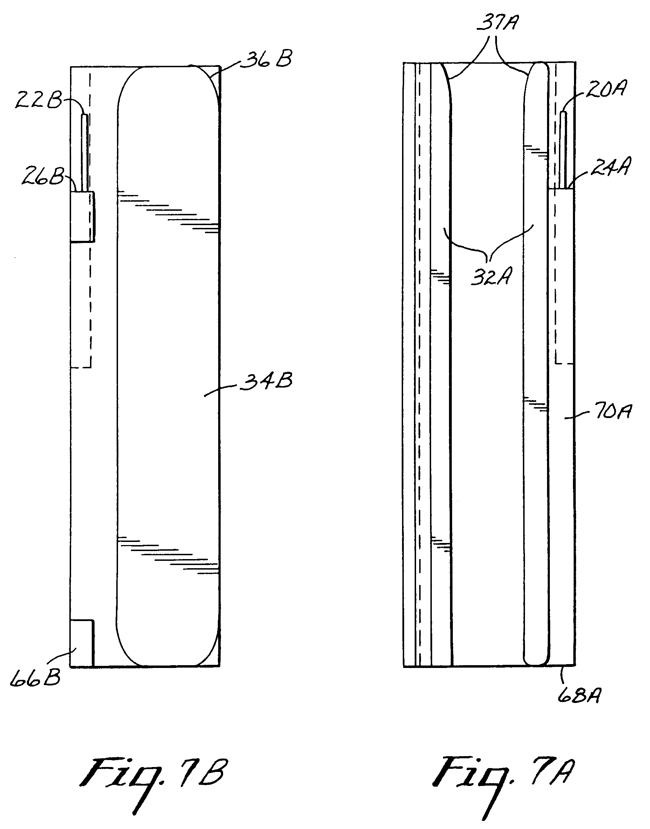

In order to facilitate mounting one module on another, the upper and/or lower ends of the male dovetail-type slide connections 34, 34A and 34B are preferably tapered as illustrated at 36B in figure 7B to ease their introduction into the open slot area of the female-type slide connections 32, 32A and 32B. The open slot area of the female dovetail-type slide connections 32, 32A and 32B may also be flared outwardly adjacent their upper and/or lower ends as illustrated at 37A in figure 7A to guide the male dovetail-type slide connections 34, 34A and 34B into the open slot area of the female slide connections 32, 32A and 32B.

The slide connections 32, 32A, 32B, 34, 34A and 34B preferably extend along a substantial portion of the entire height of the sides 28, 28A, 28B, 30, 30A or 30B of the modules 12, 14 and 16. The height of the slide connections 32, 32A, 32B, 34, 34A and 34B is most preferably at least half of the height of the module 12, 14 or 16. For example, the height of the slide connections 32, 32A, 32B, 34, 34A and 34B may be approximately two thirds of the height of the modules 12, 14 and 16. One example is slide connections 32, 32A, 32B, 34, 34A and 34B having a six inch (150mm) height on modules 12, 14 and 16 having a nine inch (230mm) height.

Preferably, each latching surface (e.g., 26 in figure 6) is formed by a shoulder (also 26) including a flange 38 adapted for engaging a latching arm 20B of a module 16 being mounted thereon. The flange 38 extends generally vertically upwardly from the shoulder 26 along an outer edge of the shoulder 26. As illustrated in figure 6, a notch 40B is provided in each latching arm 20B generally adjacent the outer end of the latching arm 20B. The notches 40B of the latching arms 20B are adapted to receive the corresponding flanges 38 of a center module 14 when the latching arms 20B and 22B are in their first position. (The notch in the opposite latching arm 22 and 22A is designated by the reference numeral 42 or 42A in figures 3 and 4.)

As illustrated in figures 7A and 7B, the shoulders 24 and 26 of the preferred embodiment are generally adjacent the upper ends of the slide connections 32, 32A, 32B, 34, 34A, and 34B. The shoulders 24 and 26 are formed in a generally upwardly-facing direction to support the latching arms 20A or 22A of adjacent modules 12 to mount the adjacent module 12 on the "center" module 14. Alternatively, it is contemplated that the shoulders and latching surfaces (not shown) could be provided adjacent the lower ends of the slide connections.

Each module 12, 14 and 16 in the preferred embodiment has a securing means generally designated 44 (figure 2) for securing the module 12, 14 or 16 to the pole 17. As illustrated in figure 2, the securing means 44 preferably comprises a generally U or J-shaped clamp bracket 46 forming a generally U or J-shaped opening for receiving the pole 17, and a movable clamp jaw member 48 mounted on one side of the bracket 46 for movement to clamp or release a pole 17 between the jaw member 48 and the opposite side of the bracket 46.

The end surface 50 of the jaw member 48 is most preferably inclined at an angle (e.g., 55-60 degrees) relative to the longitudinal axis of movement AX-2 of the jaw member 48 to apply a clamping force to the pole 17 inwardly relative to the J or U-shaped open area defined by the clamp bracket 46. The end surface 50 of the jaw member 48 may be provided with a suitable texture (e.g., a knurled or ribbed surface or rubber coating) to help secure the module 14 on the pole 17. The securing means may alternatively be of the general type shown in U.S. Design Patent No. 278,181.

Automatic means 52 is preferably provided for moving the latching arms 20 and 22 to their second position (figure 4) upon securing the module 14 to a pole 17 via the securing means 44. Most preferably, each latching arm 20 and 22 includes a rack portion 54 and 56 having gear teeth (also at 54 and 56). A gear 58 is rotatably mounted on the frame 18, with the gear teeth 60 of the gear 58 in intermeshing relationship with the gear teeth 54 and 56 of the latching arms 20 and 22. The arrangement is such that rotation of the gear 58 in one direction (counterclockwise in the drawing) withdraws the latching arms 20 and 22 from the first position (figure 4 and latching arm 20A in figure 5) to their second position (latching arms 20 and 22 in figure 5).

Biasing means (e.g., coil spring 62 or 62A) is provided for biasing the latching arms 20 and 22 or 20A and 22A toward their first position (figure 4). The biasing spring 62 or 62A preferably engages the frame 18 at one end of the spring 62 or 62A and one of the latching arms 20 or 20A to bias the latching arms 20, 20A, 20B, 22, 22A and 22B laterally outwardly relative to the module 12, 14 or 16 to their first position.

A tongue 64, 64A is provided on one of the latching arms (left latching arms 20 and 20A in the drawing). As shown in figure 2, the tongue 64, 64A or 64B extends into the securing means 44, 44A and 44B such that when the tongue 64 is engaged by a pole 17 to which the securing means 44 is secured, the tongue 64 is moved by the pole 17 to automatically move the latching arms 20 and 22 to their second position.

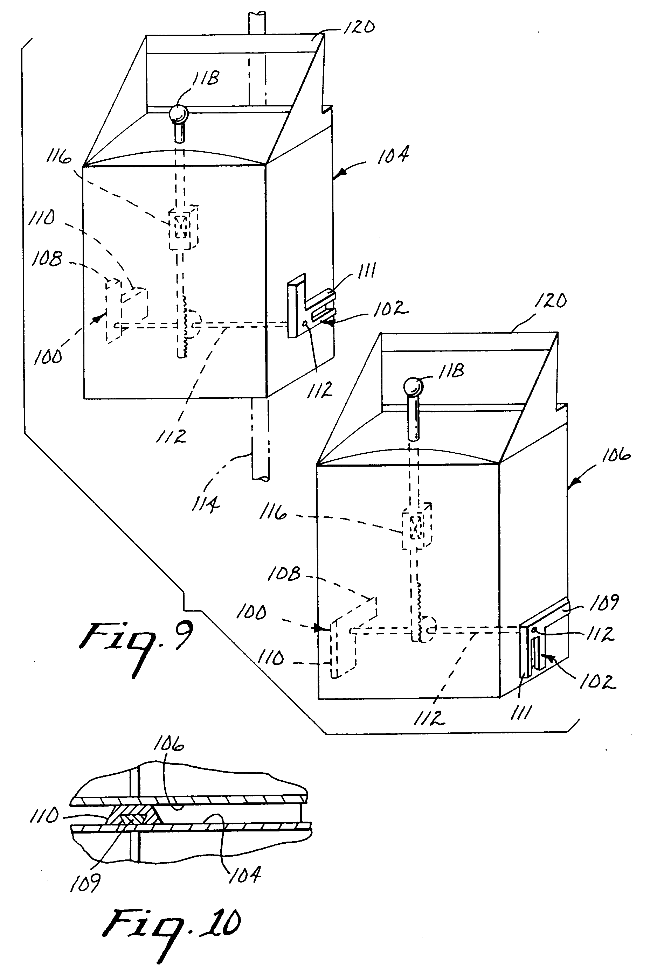

As illustrated in figure 8, when the latching arms 20A of one module 12 are extended in their first position, they will engage a blocking member 66B on another module 16 if one attempts to mount another module on a "first" side module 12. The result is that the slide connections of the attempted "second" side module cannot be inserted into the slide connections of the "first" side module 12. In other word, more than one side module is prevented from being mounted on any one side of the center module 14. The most preferred arrangement is as illustrated in the drawing where at most two side modules 12 and 16 can be mounted on a center module 14, one side module 12 or 16 on each side of the center module 14. In such an arrangement, the securing means 44 of the center module 14 will have to support at most three modules 12, 14 and 16, and the maximum load will be balanced on opposite sides of the center module 14. The bottom surface 68A on continuous member 70A illustrated in figure 7A constitutes one alternative blocking means for engaging a latching arm to block connection of a "second" side module.

The latching arms 20, 20A, 20B, 22, 22A and 22B are mounted in the frame 18, 18A or 18B for sliding movement on pins 72 and 72A in figures 3 and 4. Slots 74, 74A, 76 and 76A are provided in the latching arms 20, 20A, 20B, 22, 22A and 22B to receive the pins 72, 72A, and to allow movement of the latching arms 20, 20A, 20B, 22, 22A and 22B between their first and second positions.

One possible alternative means for moving the latching arms between their first and second conditions comprises an electrical switch arranged relative to the pole-securing bracket 46 to activate solenoid valves to withdraw the latching arms to their second condition. It is contemplated in that alternative that the solenoid valves would be of the pulse type. Other electromechanical means may also be employed to move the latching arms between their first and second positions.

Another alternative arrangement, which is not illustrated in the drawing, would be to provide downwardly-facing latching surfaces adjacent the lower ends of the slide connections, and upwardly-directed notches in the latching arms, which would also be positioned adjacent the lower end of the slide connection. In that alternative arrangement, the latching arms of a side module would be retracted in the first condition to allow the side module to be mounted on another (center) module in its second condition. The latching arms of the center module would be extended in the second condition to allow the downwardly-facing latching surface of the side module to interlock with the latching arms of the center module. In such an alternative, the latching arms would preferably be biased to their retracted position, and automatically extended to their extended position when the module is mounted on a pole stand.

The latching arms 20, 20A, 20B, 22, 22A and 22B of the preferred embodiment may be considered as latching means for mounting the module 12, 14 or 16 on another module 12, 14 or 16, and the latching surfaces or shoulders 24, 24A, 24B, 26, 26A and 26B may be considered as latch-receiving means for allowing the latching means of another module 12, 14 or 16 to be mounted on the module 12, 14 or 16. One example of a condition-setting means is the tongue 64 which automatically moves the latching means (latching arms 20 and 22) to its second, non-latching position when the module 14 is mounted on the pole 17. In the second, non-latching position, the latching arms 20 and 22 are withdrawn into the module, with the result that the module 12 will allow other modules 14 or 16 to be mounted on its slide connections.

It is also contemplated that a detent (not shown) or positive locking means may be provided to prevent removal of a "side" module from a center module. For example, a detent could be provided on the slide connections, possibly by providing a spring-loaded ball detent in one of the slide connection (e.g., the female dovetail-type slide connections), and a detent-receiving recess in the other slide connection (e.g., the male dovetail-type slide connection) to increase resistance to lifting a side module from the center module. Another example would be to provide a manually operated latch (not shown) to positively lock a side module on the center module. In that arrangement, the side module could not be removed from the center module unless a button or lever was manually operated to release the latch. Such a latch might include a spring bias to its latched position such that the latch of the side module automatically clicks into a latch-receiving recess in the center module to lock them together.

Figures 9 and 10 illustrate an alternative embodiment of the invention in which the interlocking means comprises a pair of generally L-shaped support bodies 100 and 102 rotatably mounted on opposite sides of the module 104 and 106. The same features on modules 104 and 106 are indicated by the same reference character. The modules 104 and 106 shown are "interchangeable" as defined above.

Each of the support bodies 100 and 102 has two legs 108 and 110 or 109 and 111. One leg 108, 109 comprises a male dovetail-type slide connection (also 108, 109) adapted for connection with a complementary female dovetail-type slide connection 110 or 111 of a similar module 106. The other leg 110, 111 comprising a female dovetail-type slide connection (also 110, 111) adapted for connection with a complementary male dovetail-type slide connection 108 or 109 of a similar module 104.

In the embodiment of figures 9 and 10, the condition-setting means comprises means (e.g., axle 112 shown in phantom) for pivotably mounting the support bodies 100 and 102 on opposite sides of the module 104 and 106. The male dovetail-type slide connections 108 and 109 of the support bodies 102 and 104 are aligned in one direction (upwardly in module 104 and in the backward direction in module 106 in figure 9), and the female dovetail-type slide connections 110 and 111 of the support bodies 100 and 102 are aligned in another direction (backwardly in module 104 and downwardly in module 106 in figure 9) generally perpendicular to the direction of the male dovetail-type slide connections 108 and 109.

The support bodies 100 and 102 are pivotable between a first position (module 106 in figure 9) and a second position (module 104 in figure 9). In the first position, one aligned pair of the male or female dovetail-type slide connections 110 and 111 are aligned in the vertical direction. In the second position, the other aligned pair of said male or female dovetail-type slide connections 108 and 109 are aligned in the vertical direction.

The arrangement is such that the first position of the support bodies 100 and 102 corresponds to the first condition of the interlocking means, i.e., that the support bodies 100 and 102 of "side" module 106 are oriented to mount the module 106 on a "center" module 104 but the support bodies 100 and 102 "side" module 106 will not allow a second "side" module (not shown) to be mounted on the "side" module 106. The arrangement is also such that the second position of the support bodies 100 and 102 corresponds to the second condition of the interlocking means, i.e., that the support bodies 100 and 102 of "center" module 104 are oriented to allow a "side" module 106 to be mounted on the "center" module 104.

In the particular embodiment shown in figure 9, the male dovetail-type slide connections 108 and 109 must be aligned in the vertically upward direction ("center" module 104) to allow a "side" module 106 to be mounted thereon. The female dovetail-type slide connections 110 and 111 must be aligned in the vertically downward direction ("side" module 106) to be mounted on the male dovetail-type slide connections 108 or 109 of the "center" module 104. Figure 10 is a cross-sectional illustration of a female dovetail-type slide connection 110 of module 106 mounted on a male dovetail-type slide connection 109 of module 104.

As was the case in the preferred embodiment, means (not shown) is provided for securing the module 104 or 106 on a pole 114. Biasing means generally indicated at 116 in phantom may be provided for biasing the support bodies 100 and 102 to one of their positions. The biasing means 116 shown in figure 9 can either be designed to bias the support bodies 100 and 102 to their first position (module 106) or to their second position (module 104).

The condition-setting means may comprise, in addition to an axle 112, manual or automatic means for setting the module 104 or 106 in its second condition upon securing the module 104 to a pole 114 via the securing means. Although automatic means of the general type described with respect to the embodiment of figures 1-8 is preferred, manual means such as a pull-type knob 118 are shown in figure 9 for manually moving the support bodies 100 and 102 between their first and second positions. The pull-type knob 118 may include a handle (not shown) associated with the handle 120 of the module 104 or 106 to facilitate its operation.

### OPERATION

The operation of the system 10 will be described with reference to the preferred embodiment shown in figures 1-8. The center module 14 is first mounted on a pole stand 17 by clamping the pole 17 with the movable jaw 48. As the securing bracket 46 is brought around the pole 17, the tongue 64 is engaged by the pole 17 and moved out of the open space defined by the securing bracket 46, as illustrated in figure 2 by the relative position of the tongue 64 compared to either tongue 64A or 64B of the "side" modules.

The movement of the tongue 64 by the pole 17 causes the latching arms 20 and 22 to retract into the module 14. This is illustrated by the comparison of figures 3 and 4. In figure 4, the tongue 64 has been engaged by the pole 17 to move latching arm 20 inwardly (rightwardly in figure 4), thus causing the gear 58 to rotate counterclockwise to move the other latching arm 22 inwardly (leftwardly in figure 4). The latching arms 20 and 22 are now withdrawn relative to the latching surfaces 24 and 26 and slide connections 32 and 34 to allow a slide connection of a similar module to be mounted on either/both slide connection 32 and 34. The center module 14 is now ready to have side modules 12 and/or 16 mounted thereon.

To mount the side modules 12 and/or 16 on the center module 14, the slide connections 32A, 32B, 34A or 34B of the side modules 12 or 16 are connected with the adjacent slide connection 32 or 34 of the center module 14 until the latching arm 22A or 20B engages the respective shoulder 24 or 26 of the center module 14. (The latching arms 20A, 20B, 22A and 22B of the "side" modules 12 and 16 are in their first position, in which they extend relative to the latching surfaces and slide connections of the respective side module 12 or 16 a distance sufficient to engage the shoulder 24 or 26 of a similar module 14 when one of the slide connections are mounted on the slide connection of the similar module 14.) At this point, the modules 12, 14 and 16 are properly interconnected on the pole 17.

### Avoidance of Undesired Mounting Arrangements

Mounting of more than one module on one side of a center module 14 may be considered undesirable for a number of reasons, including the possibility of excessively unbalanced loads if two or three modules are mounted on one side of the center module and no modules are mounted on the other side. It may also be desired to limit the total number of modules to be mounted at one location on a pole 17 to prevent the pole-securing means 44 of the center module 14 from being overloaded.

Such undesired arrangements are prevented by the interengagement of the extended latching arms 20A and 22B of the side modules 16 and 12 with the blocking member 66B or blocking surface 68A of the undesired "second" side module. As illustrated in figure 8, if someone were to attempt to mount side module 16 on the right side 28A of a side module 12 that is already mounted on a center module 14, the extended latching arm 20A of the "first" side module 12 would engage the blocking member 66B to prevent the slide connections 32A and 34B from being interconnected.

## Claims

1. System for interlocking a plurality of modules in side-by-side relationship, the system comprising
- a plurality of interchangeable modules (12,14,16; 104,106) including at least a first module and a second module, the modules (12,14,16;104,106) each comprising:
- a frame (18,18A,18B) having opposite sides, and
- interlocking means on the frame (18,18A,18B) adapted for attaching at least two other modules (12,14,16;104,106) of the system (10) to the module (12,14,16;104,106), the interlocking means including:
- condition-setting means for setting the interlocking means in a first condition for mounting the module on another module of the system (10) or in a second condition for mounting other module(s) of the system (10) thereon, such that when the interlocking means is set in its first condition the interlocking means is capable of being mounted on the interlocking means of another module of the system (10) that has been set in its second condition but the interlocking means of the module is not capable of having mounted thereon the interlocking means of another module of the system and when the interlocking means is set in its second condition it is capable of having mounted thereon the interlocking means of two other modules of the system (10) that have been set in their first condition.

2. System according to claim 1 further characterized in that the modules (12,14,16;104,106) are medical device modules (12,14,16;104,106).

3. System according to claim 2 further characterized in that at least one medical device module (12,14,16;104, 106) is an infusion pump.

4. System according to any one of claims 1 to 3 further characterized in that each module (12,14,16) further comprises securing means (44,44A,44B) for securing the module (12,14,16) to a pole (17), the condition-setting means of each module (12,14,16) including automatic means (52,52A,52B) for setting the module (12,14,16) in its second condition upon securing the module (12,14,16) to a pole (17) via the securing means (44,44A,44B).

5. System according to any one of claims 1 to 4 further characterized in that the interlocking means of each module (12,14,16) comprises one of each a male and a female dovetail-type slide connection, the male dovetail-type slide connection being complementary to the female dovetail-type slide connection of other modules (12,14,16) of the system (10), the male and female dovetail-type slide connections being arranged along opposite sides of each module (12,14,16) such that the direction of slide in the connection is generally along a vertical axis.

6. System according to claim 5 further characterized in that the condition-setting means of each module (12, 14,16) further comprises:
- a shoulder (24,26,24A,26B) adjacent each of the male and female dovetail-type slide connections, and
- a pair of latching arms (20,22,20A,22A,20B,22B) movable mounted on the frame for movement between a first position, corresponding to the first condition of the interlocking means, to a second position, corresponding to the second condition of the interlocking means,
- the arrangement being such that, when the interlocking means of one module (12,16) has been set in its first condition, with the latching arms of that module (12,16) thus placed in their first position:
(a) the latching arms of that module (12,16) are adapted to engage a shoulder (24,26) on another module (14), that has been placed in its second condition, when a dovetail-type slide connection of the module (12,16) is mounted on the complimentary dovetail-type slide connection on the other module (14), and
(b) the latching arms of the module (12,16) are adapted to prevent such engagement of the dovetail-type slide connections of another module (12,14,16) as would permit a latching arm of such other module (12,14,16) to engage either shoulder (24,26) on the module (12,14, 16).

7. System according to claim 6 further characterized in that
- dovetail-type slide connections (32,34,32A,34A, 32B,34B) of each module (12,14,16) have upper and lower ends relative to the intended use of the module (12,14,16), each shoulder (24,26) including a flange (38) extending generally vertically upwardly from the shoulder (26) along an outer edge of the shoulder (26), the shoulders (24,26) being generally adjacent the upper ends of the dovetail-type slide connections (32,34,32A,34A,32B,34B), and
- each latching arm (20,22,20A,22A,20B,22B) has a notch (40,42,40A,42A,40B) therein generally adjacent an outer end of the latching arm (20,22,20A, 22A,20B,22B), the notches (40,42,40A,42A,40B) of the latching arms (20,22,20A,22A,20B,22B) being adapted to receive a flange (38) of another module (12,14,16) when the latching arms (20,22,20A,22A, 20B,22B) are in their first position corresponding to the first configuration of the interlocking means.

8. System according to claim 7 further characterized in that biasing means (62,62A) is provided on each module (12,14,16) for biasing the latching arms (20,22,20A, 22A20B,22B) to their first position, and arm-with-drawing means is provided for withdrawing the latching arms (20,22,20A,22A,20B,22B) from their first position to their second position, the arrangement of the first and second positions being such that the latching arms (20,22,20A,22A,20B,22B) partially extend from the module (12,14,16) when in their first position and are withdrawn into the module (12,14,16) when in their second position.

9. System according to claim 8 further characterized in that the arm-withdrawing means of each module (12,14, 16) comprises a gear (58,58A) rotatably mounted in the frame (18,18A), and a rack (54,56,54A,56) of gear teeth on each of the latching arms (20,22,20A,22A), the latching arms (20,22,20A,22A) being slidably mounted on the frame (18,18A), with the gear teeth (54, 56,54A,56A) of the latching arms (20,22,20A,22A) engaging the gear (58,58A) in a rack-and-pinion engagement, the biasing means (62,62A) of each module (12, 14,16) comprising a spring (62,62A) engaging the frame (18,18A) and one of the latching arms (20,20A) to bias the latching arms (20,22,20A,22A,20B,22B) toward their first position.

10. System according to claim 6 or 9 further characterized in that the automatic means (52,52A,52B) of each module (12,14,16) comprises a tongue (64,64A,64B) operatively connected to at least one of the latching arms (20,20A,20B), the tongue (64,64A,64B) being positioned, relative to the securing means (44,44A,44B), for engagement by the pole (17) to move the tongue (64,64A,64B) and latching arms (20,22,20A,22A,20B, 22B) to their second position.

11. System according to any one of claims 1 to 3 further characterized in that said interlocking means of each module (104,106) comprises a pair of generally L-shaped support bodies (100,102) rotatably mounted on opposite sides of said module (104,106), each of said support bodies (100,102) having two legs (108,110, 109,111) including one leg (108,109) comprising a male dovetail-type slide connection (108,109) adapted for connection with a complementary female dovetail-type slide connection (110,111) of a similar module (104, 106), and another leg (110,111) comprising a female dovetail-type slide connection (110,111) adapted for connection with a complementary male dovetail-type slide connection (108,109) of a similar module (104, 106),
said condition-setting means comprising:
- means (112) for pivotably mounting said support bodies (100,102) on opposite sides of said module (104,106), with said male dovetail-type slide connections (108,109) of said support bodies (100,102) being aligned in one direction and said female dovetail-type slide connections (110,111) of said support bodies (100,102) being aligned in another direction generally perpendicular to the direction of said male dovetail-type slide connections (108, 109),
- said support bodies (100,102) being pivotable between a first position, wherein one aligned pair of said male or female dovetail-type slide connections (108,109,110,111) are aligned in the vertical direction, and a second position, wherein the other aligned pair of said male or female dovetail-type slide connections (108,109,110,111) are aligned in the vertical direction.

12. System according to claim 11 further characterized in that the first position of said support bodies (100, 102) corresponds to the first condition of said interlocking means and the second position of said support bodies (100,102) corresponds to the second condition of said interlocking means; said module (104,106) further comprising:
- means for securing said module (104,106) on a pole (114), and
- biasing means (116) for biasing said support bodies (104,106) to their first position,
- said condition-setting means further comprising automatic means for setting said module (104,106) in its second condition upon securing said module (104,106) to a pole (114) via the securing means.

## Patentansprüche

1. System zur gegenseitigen Verriegelung mehrerer Module in Seite-an-Seite-Anordnung zueinander, mit
- mehreren auswechselbaren Modulen (12,14,16;104,106), die mindestens ein erstes Modul und ein zweites Modul (12, 14,16;104,106) umfassen, wobei jedes Modul aufweist:
- einen Rahmen (18,18A,18B) mit einander entgegengesetzten Seiten, und
- einer an dem Rahmen (18,18A,18B) angeordneten Verriegelungseinrichtung, die in der Lage ist, mindestens zwei weitere Module (12,14,16;104,106) des Systems (10) an dem Modul (12,14,16;104,106) zu befestigen, wobei die Verriegelungseinrichtung aufweist:
- eine Zustandseinstelleinrichtung zum Einstellen der Verriegelungseinrichtung auf einen ersten Zustand zum Montieren des Moduls an einem weiteren Modul des Systems (10), oder auf einen zweiten Zustand zum Montieren eines oder mehrerer weiterer Module des Systems (10) an dem Modul, derart, daß, wenn die Verriegelungseinrichtung auf ihren ersten Zustand eingestellt ist, die Verriegelungseinrichtung sich an der Verriegelungseinrichtung eines weiteren Moduls des Systems (10) montieren läßt, die ihrerseits auf ihren zweiten Zustand eingestellt ist, die Verriegelungseinrichtung des Moduls jedoch nicht bereit ist, die Verriegelungseinrichtung eines weiteren Moduls des Systems an sich montieren zu lassen, und, wenn die Verriegelungseinrichtung auf ihren zweiten Zustand eingestellt ist, sie bereit ist, die Verriegelungseinrichtungen zweier weiterer Module des Systems (10), die ihrerseits auf ihren ersten Zustand eingestellt sind, an sich befestigen zu lassen.

2. System nach Anspruch 1, ferner dadurch gekennzeichnet, daß die Module (12,14,16;104,106) medizintechnische Module (12,14,16;104,106) sind.

3. System nach Anspruch 2, ferner dadurch gekennzeichnet, daß mindestens ein medizintechnisches Modul (12,14,16;104,106) ein Infusionspumpe ist.

4. System nach einem der Ansprüche 1 bis 3, ferner dadurch gekennzeichnet, daß jedes Modul (12,14,16) ferner eine Sicherungseinrichtung (44,44A,44B) zum Sichern des Moduls (12,14,16) an einem Ständer (17) aufweist, und daß die Zustandseinstelleinrichtung jedes Moduls (12,14,16) eine Automatik-Einrichtung (52,52A,52B) aufweist, die das Modul (12,14,16) auf seinen zweiten Zustand einstellt, wenn das Modul (12,14,16) mittels der Sicherungseinrichtung (44, 44A,44B) an dem Ständer (17) gesichert wird.

5. System nach einem der Ansprüche 1 bis 4, ferner dadurch gekennzeichnet, daß die Verriegelungseinrichtung jedes Moduls (12,14,16) jeweils eine männliche und eine weibliche Schwalbenschwanz-Schubverbindung aufweist, die männliche Schwalbenschwanz-Schubverbindung der weiblichen Schwalbenschwanz-Schubverbindunganderer Module (12,14,16) des Systems (10) komplementär ist, und die männliche und die weibliche Schwalbenschwanz-Schubverbindung entlang einander entgegengesetzter Seiten jedes Moduls (12,14,16) derart angeordnet sind, daß die Schubrichtung in der Verbindung im wesentlichen längs einer vertikalen Achse verläuft.

6. System nach Anspruch 5, ferner dadurch gekennzeichnet, daß die Zustandseinstelleinrichtung jedes Moduls (12,14,16) ferner aufweist:
- eine Schulter (24,26,24A,26B) in der Nähe der männlichen und der weiblichen Schwalbenschwanz-Schubverbindung, und
- ein Paar Verriegelungsarme (20,22,20A,22A,20B,22B), die an dem Rahmen zur Bewegung zwischen einer ersten Position, die dem ersten Zustand der Verriegelungseinrichtung entspricht, in eine zweite Position, die dem zweiten Zustand der Verriegelungseinrichtung entspricht, befestigt sind,
- wobei die Anordnung derart beschaffen ist, daß, wenn die Verriegelungseinrichtung eines Moduls (12,16) auf ihren ersten Zustand eingestellt ist und somit die Verriegelungsarme dieses Moduls (12,16) in ihrer ersten Position angeordnet sind:
(a) die Verriegelungsarme dieses Moduls (12,16) in der Lage sind, an eine Schulter (24,26) an einem weiteren Modul (14), das auf seinen zweiten Zustand eingestellt ist, anzugreifen, wenn eine Schwalbenschwanz-Schubverbindung des Moduls (12,16) an der an dem anderen Modul (14) ausgebildeten komplementären Schwalbenschwanz-Schubverbindung montiert ist, und
(b) die Verriegelungsarme des Moduls (12,16) zum Verhindern dieses durch die Schwalbenschwanz-Schubverbindungen eines weiteren Moduls (12,14,16) erfolgenden Angreifens in der Lage sind, das einem Verriegelungsarm eines solchen weiteren Moduls (12,14,16) erlauben würde, an eine der an dem Modul (12,14,16) ausgebildeten Schultern (24,26) anzugreifen.

7. System nach Anspruch 6, ferner dadurch gekennzeichnet, daß
- Schwalbenschwanz-Schubverbindungen (32,34,32A,34A,32B, 34B) jedes Moduls (12,14,16) bezogen auf den beabsichtigten Zweck des Moduls (12,14,16) obere und untere Enden aufweisen, jede Schulter (24,26) einen Flansch (38) aufweist, der sich von der Schulter (26) ausgehend im wesentlichen vertikal aufwärts längs eines Außenrandes der Schulter (26) erstreckt, die Schultern (24,26) im wesentlichen nahe den oberen Enden der Schwalbenschwanz-Schubverbindungen (32,34,32A,34A,32B,34B) angeordnet sind, und
- jeder Verriegelungsarm (20,22,20A,22A,20B,22B) eine Nut (40,42,40A,42A,40B) aufweist, die im wesentlichen nahe dem äußeren Ende des Verriegelungsarms (20,22,20A,22A, 20B,22B) verläuft, wobei die Nuten (40,42,40A,42A,40B) der Verriegelungsarme (20,22,20A,22A,20B,22B) in der Lage sind, einen Flansch (38) eines weiteren Moduls (12,14,16) aufzunehmen, wenn die Verriegelungsarme (20, 22,20A,22A,20B,22B) in ihrer ersten Position, die der ersten Konfiguration der Verriegelungseinrichtung entspricht, angeordnet sind.

8. System nach Anspruch 7, ferner dadurch gekennzeichnet, daß an jedem Modul (12,14,16) eine Vorspanneinrichtung (62, 62A) zum Vorspannen der Verriegelungsarme (20,22,20A,22A, 20B,22B) in ihre erste Position vorgesehen ist, und eine Armrückzieheinrichtung zum Zurückziehen der Verriegelungsarme (20,22,20A,22A,20B,22B) aus ihrer ersten Position in ihre zweite Position vorgesehen ist, wobei die Anordnung der ersten und der zweiten Positionen derart beschaffen ist, daß die Verriegelungsarme (20,22,20A,22A,20B,22B) in ihrer ersten Position teilweise von dem Modul (12,14,16) abstehen und in ihrer zweiten Position in das Modul (12, 14,16) zurückgezogen sind.

9. System nach Anspruch 8, ferner dadurch gekennzeichnet, daß die Armrückzieheinrichtung jedes Moduls (12,14,16) ein drehbar in dem Rahmen (18,18A) montiertes Zahnrad (58,58A) und an jedem der Verriegelungsarme (20,22,20A,22A) eine Zahnstange (54,56,54A,56A) aufweist, die Verriegelungsarme (20,22,20A,22A) gleitbar an dem Rahmen (18,18A) montiert sind, die Zahnstange (54,56,54A,56A) der Verriegelungsarme (20,22,20A,22A) in Verzahnungs-Eingriff an das Zahnrad (58,58A) angreift, und die Vorspanneinrichtung (62,62A) jedes Moduls (12,14,16) eine Feder (62,62A) aufweist, die an den Rahmen (18,18A) und einen der Verriegelungsarme (20,20A) angreift, um die Verriegelungsarme (20,22,20A, 22A,20B,22B) auf ihre erste Position hin vorzuspannen.

10. System nach Anspruch 6 oder 9, ferner dadurch gekennzeichnet, daß die Automatik-Einrichtung (52,52A,52B) jedes Moduls (12,14,16) eine betriebsmäßig mit mindestens einem der Verriegelungsarme (20,20A,20B) verbundene Zunge (64, 64A,64B) aufweist, die relativ zu der Sicherungseinrichtung (44,44A,44B) derart positioniert ist, daß der Ständer (17) an sie angreifen kann, um die Zunge (64,64A,64B) und die Verriegelungsarme (20,22,20A,22A,20B,22B) in ihre zweite Position zu bewegen.

11. System nach einem der Ansprüche 1 bis 3, ferner dadurch gekennzeichnet, daß die Verriegelungseinrichtung jedes Moduls (104,106) ein Paar im wesentlichen L-förmiger Haltekörper (100,102) aufweist, die drehbar an einander entgegengesetzten Seiten des Moduls (104,106) befestigt sind, wobei jeder der Haltekörper (100,102) zwei Schenkel (108, 110,109,111) aufweist, von denen ein Schenkel (108,109) eine männliche Schwalbenschwanz-Schubverbindung (108,109), die mit einer komplementären weiblichen Schwalbenschwanz-Schubverbindung (110,111) eines gleichartigen Moduls (104,106) verbindbar ist, und ein weiterer Schenkel (110, 111) eine weibliche Schwalbenschwanz-Schubverbindung (110,111) aufweist, die mit einer komplementären männlichen Schwalbenschwanz-Schubverbindung (108,109) eines gleichartigen Moduls (104,106) verbindbar ist,
wobei die Zustandseinstelleinrichtung aufweist:
- eine Einrichtung (112) zur schwenkbaren Befestigung der Haltekörper (100,102) an entgegengesetzten Seiten des Moduls (104,106), wobei die männlichen Schwalbenschwanz-Schubverbindungen (108,109) der Haltekörper (100,102) in einer Richtung ausgerichtet sind und die weiblichen Schwalbenschwanz-Schubverbindungen (110,111) der Haltekörper (100,102) in einer anderen Richtung ausgerichtet sind, die im wesentlichen rechtwinklig zu der Richtung der männlichen Schwalbenschwanz-Schubverbindungen (108, 109) verläuft,
- wobei die Haltekörper (100,102) schwenkbar sind zwischen einer ersten Position, in der ein ausgerichtetes Paar der männlichen oder weiblichen Schwalbenschwanz-Schubverbindungen (108,109,110,111) in der vertikalen Richtung ausgerichtet ist, und einer zweiten Position, in der das andere ausgerichtete Paar der männlichen oder weiblichen Schwalbenschwanz-Schubverbindungen (108,109, 110,111) in der vertikalen Richtung ausgerichtet ist.

12. System nach Anspruch 11, ferner dadurch gekennzeichnet, daß die erste Position der Haltekörper (100,102) dem ersten Zustand der Verriegelungseinrichtung entspricht und die zweite Position der Haltekörper (100,102) dem zweiten Zustand der Verriegelungseinrichtung entspricht, und das Modul (104,106) ferner aufweist:
- eine Einrichtung zum Sichern des Moduls (104,106) an einem Ständer (114), und
- eine Vorspanneinrichtung (116) zum Vorspannen der Haltekörper (100,102) auf ihre erste Position hin,
- wobei die Zustandseinstelleinrichtung ferner eine Automatik-Einrichtung aufweist, die das Modul (104,106) auf seinen zweiten Zustand einstellt, wenn das Modul (104, 106) mittels der Sicherungseinrichtung an einem Ständer (114) gesichert ist.

## Revendications

1. Système pour verrouiller mutuellement plusieurs modules côte à côte, le système comportant :
- plusieurs modules interchangeables (12, 14, 16; 104, 106) comportant au moins un premier module et un deuxième module, les modules (12, 14, 16; 104, 106) comportant chacun :
- un châssis (18, 18A, 18B) ayant des côtés opposés, et
- des moyens, de verrouillage mutuel situés sur le châssis (18, 18A, 18B) adaptés pour fixer au moins deux autres modules (12, 14, 16; 104, 106) du système (10) sur le module (12, 14, 16; 104, 106), les moyens de verrouillage mutuel comportant :
- des moyens de mise en état destinés à mettre les moyens de verrouillage mutuel dans un premier état pour permettre le montage du module sur un autre module du système (10) ou dans un second état pour permettre le montage d'un autre ou d'autres modules du système (10) sur celui-ci, de telle sorte que lorsque les moyens de verrouillage mutuel sont mis dans leur premier état, les moyens de verrouillage mutuel sont capables d'être montés sur les moyens de verrouillage mutuel d'un autre module du système (10) qui ont été mis dans leur second état mais les moyens de verrouillage mutuel du module ne sont pas capables de recevoir les moyens de verrouillage mutuel d'un autre module du système et lorsque les moyens de verrouillage mutuel sont mis dans leur second état, ils sont capables de recevoir les moyens de verrouillage mutuel de deux autres modules du système (10) qui ont été mis dans leur premier état.

2. Système selon la revendication 1, caractérisé en outre en ce que les modules (12, 14, 16; 104, 106) sont des modules formant dispositif médical (12, 14, 16; 104, 106).

3. Système selon la revendication 2, caractérisé en outre en ce qu'au moins un module formant dispositif médical (12, 14, 16; 104, 106) est une pompe d'injection.

4. Système selon l'une quelconque des revendications 1 à 3, caractérisé en outre en ce que chaque module (12, 14, 16) comporte en outre des moyens de fixation (44, 44A, 44B) pour fixer le module (12, 14, 16) sur un poteau (17), les moyens de mise en état de chaque module (12, 14, 16) comportant des moyens automatiques (52, 52A, 52B) pour mettre le module (12, 14, 16) dans son second état lors de la fixation du module (12, 14, 16) sur un poteau (17) via les moyens de fixation (44, 44A, 44B).

5. Système selon l'une quelconque des revendications 1 à 4, caractérisé en outre en ce que les moyens de verrouillage mutuel de chaque module (12, 14, 16) comportent une liaison parmi une liaison coulissante du type queue d'aronde mâle et une liaison coulissante du type queue d'aronde femelle, la liaison coulissante du type queue d'aronde mâle étant complémentaire de la liaison coulissante du type queue d'aronde femelle des autres modules (12, 14, 16) du système (10), les liaisons coulissantes du type queue d'aronde mâle et femelle étant agencées le long de côtés opposés de chaque module (12, 14, 16) de telle sorte que la direction de coulissement dans la liaison est de manière générale située le long d'un axe vertical.

6. Système selon la revendication 5, caractérisé en outre en ce que les moyens de mise en état de chaque module (12, 14, 16) comportent en outre :
- un épaulement (24, 26, 24A, 26B) adjacent à chacune des liaisons coulissantes du type queue d'aronde mâle et femelle, et
- une paire de bras de verrouillage (20, 22, 20A, 22A, 20B, 22B) montés de manière mobile sur le châssis pour se déplacer entre une première position, correspondant au premier état des moyens de verrouillage mutuel, et une seconde position, correspondant au second état des moyens de verrouillage mutuel,
- l'agencement étant tel que lorsque les moyens de verrouillage mutuel d'un premier module (12, 16) sont établis dans leur premier état, les bras de verrouillage de ce module (12, 16) étant donc placés dans leur première position :
(a) les bras de verrouillage de ce module (12, 16) sont adaptés pour venir en contact avec un épaulement (24, 26) situé sur un autre module (14), qui a été placé dans son second état, lorsqu'une liaison coulissante du type queue d'aronde du module (12, 16) est montée sur la liaison coulissante du type queue d'aronde complémentaire située sur l'autre module (14), et
(b) les bras de verrouillage du module (12, 16) sont adaptés pour empêcher une telle prise des liaisons coulissantes du type queue d'aronde d'un autre module (12, 14, 16) lorsqu'est permis à un bras de verrouillage d'un tel autre module (12, 14, 16) de venir en contact avec l'un ou l'autre épaulement (24, 26) situé sur le module (12, 14, 16).

7. Système selon la revendication 6, caractérisé en outre en ce que :
- les liaisons coulissantes du type queue d'aronde (32, 34, 32A, 34A, 32B, 34B) de chaque module (12, 14, 16) ont des extrémités supérieures et inférieures par rapport à l'utilisation prévue du module (12, 14, 16), chaque épaulement (24, 26) comportant un rebord (38) s'étendant de manière générale verticalement vers le haut à partir de l'épaulement (26) le long d'un bord extérieur de l'épaulement (26), les épaulements (24, 26) étant de manière générale adjacents aux extrémités supérieures des liaisons coulissantes du type queue d'aronde (32, 34, 32A, 34A, 32B, 34B), et chaque bras de verrouillage (20, 22, 20A, 22A, 20B, 22B) a une encoche (40, 42, 40A, 42A, 40B) agencée dans celui-ci de manière générale adjacente à une extrémité extérieure du bras de verrouillage (20, 22, 20A, 22A, 20B, 22B), les encoches (40, 42, 40A, 42A, 40B) des bras de verrouillage (20, 22, 20A, 22A, 20B, 22B) étant adaptées pour recevoir un rebord (38) d'un autre module (12, 14, 16) lorsque les bras de verrouillage (20, 22, 20A, 22A, 20B, 22B) sont dans leur première position correspondant à la première configuration des moyens de verrouillage mutuel.

8. Système selon la revendication 7, caractérisé en outre en ce que des moyens de rappel (62, 62A) sont agencés sur chaque module (12, 14, 16) pour rappeler les bras de verrouillage (20, 22, 20A, 22A, 20B, 22B) vers leur première position, et des moyens de retrait des bras sont agencés pour retirer les bras de verrouillage (20, 22, 20A, 22A, 20B, 22B) à partir de leur première position vers leur seconde position, l'agencement des première et seconde positions étant tel que les bras de verrouillage (20, 22, 20A, 22A, 20B, 22B) s'étendent partiellement à partir du module (12, 14, 16) lorsqu'ils sont dans leur première position et sont retirés à l'intérieur du module (12, 14, 16) lorsqu'ils sont dans leur seconde position.

9. Système selon la revendication 8, caractérisé en outre en ce que les moyens de retrait des bras de chaque module (12, 14, 16) comportent un engrenage (58, 58A) monté de manière rotative dans le châssis (18, 18A) et une crémaillère (54, 56, 54A, 56A) constituée de dents agencées sur chacun des bras de verrouillage (20, 22, 20A, 22A), les bras de verrouillage (20, 22, 20A, 22A) étant montés de manière coulissante sur le châssis (18, 18A), les dents (54, 56, 54A, 56A) des bras de verrouillage (20, 22, 20A, 22A) venant en prise avec l'engrenage (58, 58A) selon une prise du type crémaillère et pignon, les moyens de rappel (62, 62A) de chaque module (12, 14, 16) comportant un ressort (62, 62A) venant en contact avec le châssis (18, 18A) et avec l'un des bras de verrouillage (20, 20A) pour rappeler les bras de verrouillage (20, 22, 20A, 22A, 20B, 22B) en direction de leur première position.

10. Système selon la revendication 6 ou 9, caractérisé en outre en ce que les moyens automatiques (52, 52A, 52B) de chaque module (12, 14, 16) sont constitués d'une languette (64, 64A, 64B) reliée de manière opérationnelle à au moins un des bras de verrouillage (20, 20A, 20B), la languette (64, 64A, 64B) étant positionnée, par rapport aux moyens de fixation (44, 44A, 44B), pour venir en contact avec le poteau (17) pour déplacer la languette (64, 64A, 64B) et les bras de verrouillage (20, 22, 20A, 22A, 20B, 22B) vers leur seconde position.

11. Système selon l'une quelconque des revendications 1 à 3, caractérisé en outre en ce que lesdits moyens de verrouillage mutuel de chaque module (104, 106) sont constitués de deux corps de support ayant la forme de manière générale d'un L (100, 102) montés de manière rotative sur des côtés opposés dudit module (104, 106) chacun desdits corps de support (100, 102) ayant deux pattes (108, 110, 109, 111) comportant une première patte (108, 109) constituant une liaison coulissante du type queue d'aronde mâle (108, 109) adaptée pour être reliée à une liaison coulissante du type queue d'aronde femelle complémentaire (110, 111) d'un module similaire (104, 106), et une autre patte (110, 111) constituant une liaison coulissante du type queue d'aronde femelle (110, 111) adaptée pour être reliée à une liaison coulissante du type queue d'aronde mâle complémentaire (108, 109) d'un module similaire (104, 106),
lesdits moyens de mise en état comportant :
- des moyens (112) pour monter de manière pivotante lesdits corps de support (100, 102) sur des côtés opposés dudit module (104, 106), lesdites liaisons coulissantes du type queue d'aronde mâle (108, 109) desdits corps de support (100, 102) étant alignées dans une direction et lesdites liaisons coulissantes du type queue d'aronde femelle (110, 111) desdits corps de support (100, 102) étant alignées dans une autre direction de manière générale perpendiculaire à la direction desdites liaisons coulissantes du type queue d'aronde mâle (108, 109),
- lesdits corps de support (100, 102) pouvant pivoter entre une première position, dans laquelle une paire alignée desdites liaisons coulissantes du type queue d'aronde mâle ou femelle (108, 109, 110, 111) est alignée dans la direction verticale, et une seconde position, dans laquelle l'autre paire alignée desdites liaisons coulissantes du type queue d'aronde mâle ou femelle (108, 109, 110, 111) est alignée dans la direction verticale.

12. Système selon la revendication 11, caractérisé en outre en ce que la première position desdits corps de support (100, 102) correspond au premier état desdits moyens de verrouillage mutuel et la seconde position desdits corps de support (100, 102) correspond au second état desdits moyens de verrouillage mutuel, ledit module (104, 106) comportant en outre :
- des moyens pour fixer ledit module (104, 106) sur un poteau (114), et
- des moyens de rappel (116) pour rappeler lesdits corps de support (100, 102) vers leur première position,
- lesdits moyens de mise en état comportant en outre des moyens automatiques pour mettre ledit module (104, 106) dans son second état lors de la fixation dudit module (104, 106) sur un poteau (114) via les moyens de fixation.
